(19) Europäisches Patentamt
European Patent Office
Office européen des brevets

(11) Veröffentlichungsnummer: **0 066 246**
**A2**

(12) **EUROPÄISCHE PATENTANMELDUNG**

(21) Anmeldenummer: 82104575.4

(22) Anmeldetag: 26.05.82

(51) Int. Cl.³: C 07 C 120/04
C 07 C 121/75

(30) Priorität: 02.06.81 DE 3121766

(43) Veröffentlichungstag der Anmeldung:
08.12.82 Patentblatt 82/49

(84) Benannte Vertragsstaaten:
AT BE CH DE FR GB IT LI NL SE

(71) Anmelder: BASF Aktiengesellschaft
Carl-Bosch-Strasse 38
D-6700 Ludwigshafen(DE)

(72) Erfinder: Seitz, Werner, Dr.
Bismarckstrasse 22B
D-6831 Plankstadt(DE)

(72) Erfinder: Scheib, Klaus, Dr.
Duerkheimer Strasse 7
D-6701 Schauernheim(DE)

(72) Erfinder: Michel, Alfred
Gruenstadter Strasse 52
D-6753 Enkenbach/Alsenborn(DE)

(54) **Verfahren zur Herstellung basisch substituierter Phenylacetonitrile.**

(57) Die vorliegende Erfindung betrifft ein Verfahren zur Herstellung von basisch substituierten Phenylacetonitrilen der Formel

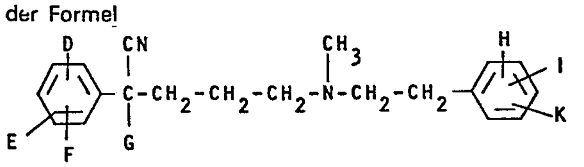
1

worin

D, E, F, G, H, I und K die in der Beschreibung angegebene Bedeutung besitzen, durch Umsetzen der entsprechenden Phenylacetonitrile mit Verbindungen der Formel

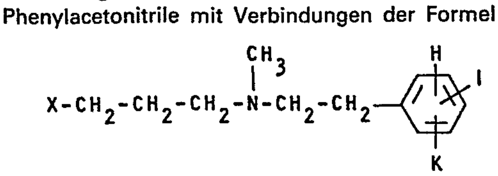
111

worin

X, H, I und K die in der Beschreibung angegebene Bedeutung besitzen in einem fest-flüssig-Phasensystem in Gegenwart eines Phasentransfer-Katalysators.

EP 0 066 246 A2

# Verfahren zur Herstellung basisch substituierter Phenylacetonitrile

Die Erfindung betrifft ein Verfahren zur Herstellung basisch substituierter Phenyacetonitrile.

Basisch substituierte Phenylacetonitrile der Formel

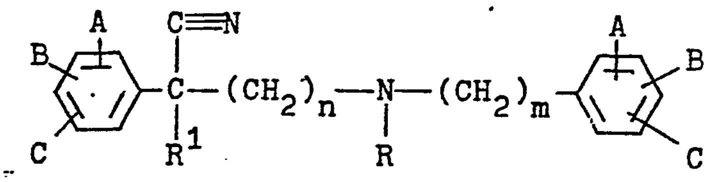

in welcher A, B und C Wasserstoff- oder Halogenatome, niedermolekulare Alkyl- bzw. Alkoxygruppen, wobei in letzterem Falle zwei benachbarte Gruppen auch gemeinsam eine Methylendioxygruppe bilden können, R einen niedermolekularen aliphatischen Rest, $R^1$ einen niedermolekularen Alkylrest, einen gesättigten oder ungesättigten cyclischen oder bicyclischen Kohlenwasserstoffrest oder die Benzyl- bzw. Phenylgruppe, n die Zahl 2, 3 oder 4 und m die Zahl 1, 2 oder 3 bedeuten sind bereits an der DE-PS 1 154 810 als Substanzen mit coronarerweiternder Wirksamkeit bekannt.

Die Verbindungen werden u.a. hergestellt, indem man in Gegenwart basischer Kondensationsmittel Phenylacetonitrile der Formel

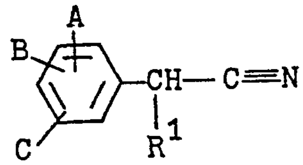

mit einer Verbindung der allgemeinen Formel

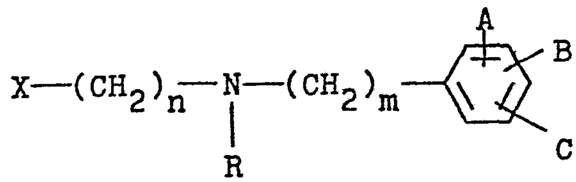

wobei A, B, C, R, $R^1$, n und m die obige Bedeutung haben und X einen reaktionsfähigen Säurerest darstellt, umsetzt. Für diese Umsetzung wird als basisches Kondensationsmittel ausschließlich Natriumamid verwendet. Andere basische Kondensationsmittel, die auf metallorganischer Basis beruhen, z.B. Butyllithium, Lithiumdialkylamide lassen sich ebenfalls mit Erfolg einsetzen (D.S. Watt, Tetrahedron Letters 1974 (9), 707-710). Natriumamid und metallorganische Kondensationsmittel weisen jedoch verschiedene Mängel auf:

1. Die Kondensationsmittel sind infolge ihrer großen Empfindlichkeit gegenüber Feuchtigkeit und ihrer leichten Entflammbarkeit sehr schwer zu handhaben. Das erfordert besonders im technischen Maßstab einen hohen sicherheitstechnischen Aufwand.

2. Die erforderlichen basischen Kondensationsmittel sind relativ teuer.

3. Die Umsetzungen müssen in absoluten Lösungsmitteln und unter einem Schutzgas ausgeführt werden.

4. Die Verwendung von Natriumamid erfordert eine Reaktionszeit von 5 bis 6 Stunden und eine Reaktionstemperatur von 110°C. Dies bedeutet einen größeren Energieaufwand und längere Belegzeiten als beim gefundenen Verfahren.

5. Bei Verwendung von Natriumamid, Natriumhydrid bzw. metallorganischer Reagenzien entstehen als Reaktionsproprodukte Ammoniak, Wasserstoff bzw. Kohlenwasserstoffe. Diese stellen ein Sicherheitsrisiko bzw. eine Umweltbelastung dar.

Analoges gilt für die weiteren bekannten Verfahren zur Herstellung der genannten Verbindungen (DE-PS 1 158 083, DE-OS 20 59 985, DE-OS 22 63 527, DE-OS 26 31 222).

Es wurde nun ein neues Verfahren zur Herstellung der genannten und ähnlicherVerbindungen gefunden, welches erhebliche Vorteile gegenüber dem geschilderten Verfahren besitzt.

Gegenstand der Erfindung ist ein Verfahren zur Herstellung basisch substituierter Phenylacetonitrile der Formel I

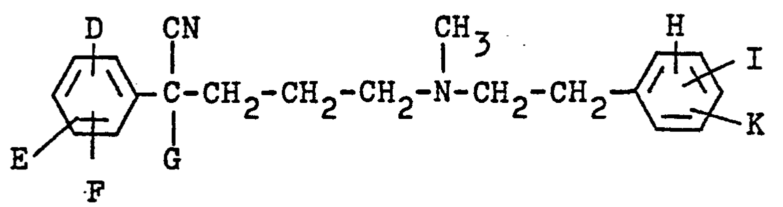

I

,

worin

D, E, F, H, I, K Wasserstoff- oder Halogenatome oder Alkoxy- oder Alkylgruppen mit 1 bis 4 Kohlenstoffatomen, G einen linearen oder verzweigten aliphatischen Kohlenwasserstoffrest mit bis zu 20 Kohlenstoffatomen, einen gesättigten oder ungesättigten cyclischen oder bicyclischen Kohlenwasserstoff mit 3 bis 20 Kohlenstoffatomen darstellen, durch Umsetzung, eines alpha-substituierten Phenylacetonitrils der Formel II

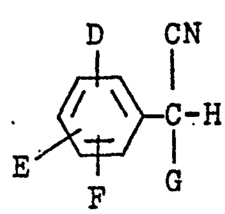

II,

worin

D, E, F und G die oben genannte Bedeutung besitzen, mit einer Verbindung der Formel III

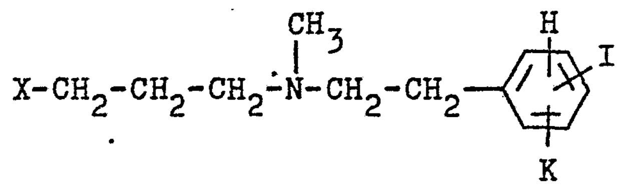

III

worin

H, I und K die oben genannte Bedeutung besitzen und X Chlor, Brom oder eine Austrittsgruppe darstellt, dadurch gekennzeichnet, daß man die Umsetzung in einem fest-flüssig-Phasensystem in Gegenwart eines Phasentransfer-Katalysators durchführt.

Als flüssige Phase für die Reaktion haben sich vor allem aromatische Kohlenwasserstoffe, wie Benzol, Toluol oder Xylole, bewährt, jedoch sind auch höhersiedende aliphatische Ether geeignet, z.B. Dioxan, Tetrahydrofuran oder Dibutylether.

Als geeignete feste Phase erwies sich insbesondere Kaliumhydroxidpulver. Hiervon werden mindestens drei Äquivalente für die Umsetzung benötigt.

Natriumhydroxidpulver ist für dieses Verfahren als feste Phase ungeeignet, da es lange Reaktionszeiten erfordert, zu starker Nebenprodukt-Bildung und damit zu einer unbefriedigenden Ausbeute führt.

Die gleichen Nachteile treten beim Arbeiten nach einem flüssig-flüssig Verfahren auf, wobei als flüssige Phasen hochkonzentrierte wäßrige Lösungen von Natrium- oder Kaliumhydroxid und toluolische Lösungen beider Reaktionspartner eingesetzt wurden.

Als Katalysatoren sind symmetrische quartäre Tetraalkylammonium- bzw. -phosphoniumsalze oder Kronenether geeignet. Beispiele hierfür sind Tetrabutylammonium-hydrogensulfat, -bromid, -iodid, -chlorid, 18-Krone-6, Dibenzo-18-Krone-6, Tetrabutylphosphonium-bromid und -chlorid. Besonders geeignet sind Iodide.

In der Formel III kann X neben Chlor oder Brom eine Austrittsgruppe darstellen. Als Austrittgruppen kommen insbesondere Mesylat, Tosylat und Triflat ($CF_3SO_2$-) in Frage.

Die Reaktion kann in einem Temperaturbereich von 50° bis 110°C durchgeführt werden. Die höchsten Ausbeuten und die geringste Bildung von Nebenprodukten werden erzielt, wenn man bei etwa 85 bis 95°C arbeitet. Dabei ist eine Reaktionszeit von etwa drei Stunden erforderlich.

Das erfindungsgemäße Verfahren besitzt nicht die oben geschilderten Nachteile des bekannten Verfahrens und liefert Ausbeuten von 85 bis 90 % eines sehr reinen Produktes. Diese Werte liegen weit über denen, die bislang für die Herstellung der genannten Verbindungen erhalten wurden. Darüber hinaus ist das neue Verfahren in seiner Durchführung wesentlich einfacher als alle bekannten Verfahren.

## Beispiel 1

alpha-Isopropyl-alpha-[N-methyl-N-homoveratryl)-amino-gamma-propyl]-3.4-dimethoxyacetonitril

In einem Dreihalskolben, der mit Rührwerk, Tropftrichter und Rückflußkühler ausgerüstet ist, werden 164 g (0,75 Mol) alpha-Isopropylveratrylcyanid in 100 ml Toluol bei 40°C gelöst. Zu dieser Lösung gibt man 195 g technisches Kaliumhydroxidpulver und 1,5 g Tetrabutylammoniumiodid. Anschließend wird unter Rühren innerhalb 45 Min. eine Lösung von 196 g (0,75 Mol) (N-Methyl-N-homoveratryl)-amino-gamma-chlorpropan in 150 ml Toluol in dem Maße zugegeben, daß die Reaktionstemperatur 90°C nicht übersteigt. Nach beendeter Zugabe wird bei 90°C 2,5 Stunden nachgerührt. Das erkaltete Reaktionsgemisch wird mit 500 ml Wasser versetzt, von der Toluolphase getrennt und diese mehrmals mit Wasser gewaschen. Nach Abziehen des Lösungsmittels erhält man 350 g Rohprodukt als gelbes Öl. Dieses wird in 700 ml Isopropanol gelöst und mit 6 M isopropanolischer Salzsäure unter Rühren versetzt. Nach 20 Stunden werden 325 g (88 %) Hydrochlorid isoliert, F = 141° bis 144°C.

Beispiel 2

alpha-(n-Dodecyl)-alpha-[N-methyl-N-homoveratryl)-amino--gamma-propyl]-3.4-dimethoxyacetonitril

In gleicher Weise wie in Beispiel 1 beschrieben werden 34,5 g alpha-Dodecyl-veratrylcyanid in 15 ml Toluol gelöst. Zu dieser Lösung gibt man 26 g technisches Kaliumhydroxidpulver, 0,2 g Tetrabutylammoniumiodid und anschließend eine Lösung von 27 g (N-Methyl-N-homoveratryl)-amino--gamma-chlorpropan in 20 ml Toluol. Nach Aufarbeitung erhält man 55 g (95 %) Rohbase als gelbes Öl. Dessen Hydrogenoxalat schmilzt bei F = 93 bis 96°C (Ether).

Beispiel 3

alpha-Isopropyl-alpha-[(N-methyl-N-homoveratryl)-amino--gamma-propyl]-3.4.5-trimethoxyphenylacetonitril

Wie in Beispiel 1 und 2 beschrieben, werden aus 24,9 g (0,1 Mol) alpha-Isopropyl-3.4.5-trimethoxyphenylacetoni-

tril, 27 g (N-Methyl-N-homoveratryl)-amino-gamma-chlorpropan, 26 g technischem KOH und 0,2 g Tetrabutylammoniumiodid 43 g (90 %) Rohbase als gelbes Öl erhalten. Daraus werden durch Lösen in Isopropanol und Ausfällen mit HCl 44 g (85 %) Hydrochlorid, F = 145-148°C isoliert.

Patentansprüche

1. Verfahren zur Herstellung basisch substituierter Phenylacetonitrile der Formel I

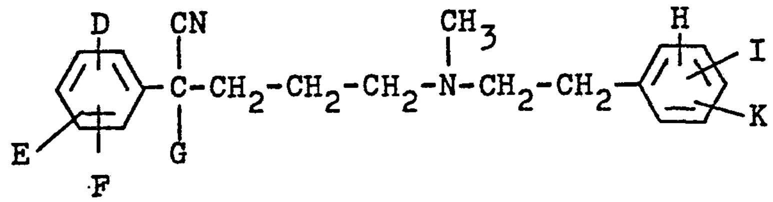

I

worin
D, E, F, H, I, K Wasserstoff- oder Halogenatome oder Alkoxy- oder Alkylgruppen mit 1 bis 4 Kohlenstoffatomen, G einen linearen oder verzweigten aliphatischen Kohlenwasserstoffrest mit bis zu 20 Kohlenstoffatomen, einen gesättigten oder ungesättigten cyclischen oder bicyclischen Kohlenwasserstoff mit 3 bis 20 Kohlenstoffatomen darstellen, durch Umsetzung eines alpha-substituierten Phenylacetonitrils der Formel II

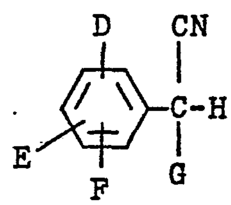

II,

worin
D, E, F und G die oben genannte Bedeutung besitzen, mit einer Verbindung der Formel III,

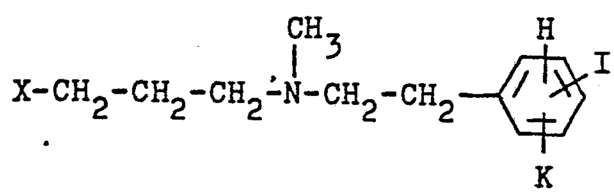

III

worin
H, I und K die oben genannte Bedeutung besitzen und X Chlor, Brom oder eine Austrittsgruppe darstellt, dadurch gekennzeichnet, daß man die Umsetzung in einem

fest-flüssig-Phasensystem in Gegenwart eines Phasentransfer-Katalysators durchführt.

2. Verfahren gemäß Anspruch 1, dadurch gekennzeichnet, daß man als feste Phase des fest-flüssigen Phasensystems Kaliumhydroxid verwendet.